(19) **Europäisches Patentamt**
**European Patent Office**
**Office européen des brevets**

(11) **EP 2 674 873 A2**

(12) **EUROPÄISCHE PATENTANMELDUNG**

(43) Veröffentlichungstag:
**18.12.2013 Patentblatt 2013/51**

(51) Int Cl.:
*G06F 17/10* (2006.01)   *G06F 17/50* (2006.01)

(21) Anmeldenummer: **12193010.1**

(22) Anmeldetag: **16.11.2012**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **12.06.2012 EP 12171633**

(71) Anmelder: **Siemens Aktiengesellschaft
80333 München (DE)**

(72) Erfinder:
• **Kohler, Dominic
80804 München (DE)**
• **Wever, Utz
81539 München (DE)**

(54) **Kontaminationsbestimmung in einem Trinkwassernetz**

(57)    Ein Verfahren zur Bestimmung eines Gefährdungspotentials durch Wasser in einem kontaminierten Leitungsnetz umfasst Schritte des Erfassens eines partiellen Differenzialgleichungssystems zur Beschreibung einer Entwicklung einer Kontamination im Leitungsnetz, des Diskretisierens des Differenzialgleichungssystems in Ort und Zeit sowie des Diskretisierens des zugeordneten Phasenraums in Zustände, wobei jedem Zustand ein Gefährdungspotential zugeordnet ist, ferner des Bestimmens von Übergangswahrscheinlichkeiten zwischen Zuständen des diskretisierten Phasenraums, des Erfassens einer ersten Kontamination zu einer ersten Zeit an einem ersten Ort des Leitungsnetzes, des Bestimmens einer Ungenauigkeit der Erfassung der Kontamination, des Bestimmens einer ersten Wahrscheinlichkeitsverteilung von Zuständen des diskretisierten Phasenraums, die der ersten Kontamination und der Unsicherheit zugeordnet ist, des Bestimmens einer zweiten Wahrscheinlichkeitsverteilung, die zeitlich zu der ersten Wahrscheinlichkeitsverteilung versetzt ist, auf der Basis der Übergangswahrscheinlichkeiten, und des Bereitstellens der zweiten Wahrscheinlichkeitsverteilung.

## FIG 1

**Beschreibung**

Die vorliegende Erfindung betrifft eine Technik zur Bestimmung einer Kontamination in einem Trinkwassernetz. Insbesondere betrifft die Erfindung eine Technik zur Bestimmung einer Ausbreitung einer Kontamination in einem Trinkwassernetz.

In Wasserversorgungs- und Entsorgungssystemen werden Modellrechnungen betrieben, um die Fortschreitung einer bereits eingetretenen Kontamination vorherzusagen, um Personaltrainings durchzuführen, um eine Netzplanung zu unterstützen, einen Versorgungs- bzw. Entsorgungsbetrieb zu erleichtern, geeignete Maßnahmen im Fall einer Kontamination zu bewerten und um eine verbesserte Platzierung von Sensoren zur Bestimmung einer Kontamination zu erzielen.

Um beispielsweise eine Ausbreitung der Kontamination im Trinkwassernetz zu modellieren, muss in der Regel von ungenauen Eingangsinformationen ausgegangen werden. Beispielsweise können die Kontaminationsquelle, die Konzentrationen, die Messzeitpunkte und die Messorte des kontaminierenden Stoffs nicht genau bestimmt sein. Darüber hinaus können Fehlmessungen vorliegen, die vom wahren Wert um mehrere Größenordnungen abweichen können. Außerdem kann der kontaminierte Stoff im Trinkwassernetz chemisch reagieren, wobei beteiligte Reaktionsparameter häufig nur sehr ungenau bekannt sind.

Die Bestimmung der Ausbreitung der Kontamination erfordert üblicherweise nur minimalistische Ausgabeparameter. In der Regel ist lediglich interessant, wie groß eine Gefährdung einer Person durch Entnahme von Wasser aus dem kontaminierten Netz an einer vorbestimmten Stelle und zu einem vorbestimmten Zeitpunkt ist. Dazu ist es üblich, Konzentrationen des Stoffs mittels des Modells zu bestimmen bzw. vorherzusagen und anschließend diskrete Gefährdungspotentiale auf der Basis der Konzentrationen zu bestimmen.

Zur Bestimmung der Ausbreitung der Kontamination in einem Leitungsnetz sind unterschiedliche Vorschläge gemacht worden. Beispielsweise existieren kommerzielle Simulationsmodelle in Form von Computerprogrammen oder Simulationspaketen, die üblicherweise eine deterministische Auswertung anbieten. Solche Modelle werden unter Anderem von Siemens im Rahmen der SIWA (Siemens Water) Produktfamilie angeboten. Andere Simulationspakete stammen beispielsweise von US EPA (United States Environmental Protection Agency) oder sind Teil einer größeren Softwarefamilie des US Homeland Security Research. Nachteilig bei der deterministischen Auswertung ist, dass Unsicherheiten von bestimmten Kontaminationen üblicherweise nicht berücksichtigt werden und diskrete Gefährdungspotentiale der Verarbeitung üblicherweise nicht zugrunde liegen. Ein numerischer Aufwand kann bei diesem Ansatz groß sein.

Andere Ansätze beschreiben die Ausbreitung von Unsicherheiten. Dazu gehören Monte Carlo Methoden, das Itö/Stratonowich Kalkül, das Polynomiale Chaos und die Unsicherheitsausbreitung. Nachteilig an diesen Ansätzen ist, dass zur Bestimmung üblicherweise eine genaue Anfangsverteilung des kontaminierenden Stoffs im Leitungsnetz erforderlich ist, dass der Rechenaufwand wesentlich höher als für eine deterministische Lösung ist und dass eine Simulation für viele Orte, viele Zeiten oder eine feine Granularität rasch die verfügbaren Rechenressourcen übersteigt.

Es ist Aufgabe der Erfindung, eine verbesserte Technik zur Bestimmung einer Ausbreitung eines kontaminierenden Stoffs in einem Leitungsnetz bereitzustellen, die mit überschaubaren Rechenressourcen auskommt und robust gegenüber Ungenauigkeiten und Fehlern ist. Die Erfindung löst diese Aufgabe mittels eines Verfahrens und eines Computerprogrammprodukts mit den Merkmalen der unabhängigen Ansprüche. Unteransprüche geben bevorzugte Ausführungsformen wieder.

Ein Verfahren zur Bestimmung eines Gefährdungspotentials durch Wasser in einem kontaminierten Leitungsnetz umfasst Schritte des Erfassens eines partiellen Differenzialgleichungssystems zur Beschreibung einer Entwicklung einer Kontamination im Leitungsnetz, des Diskretisierens des Differenzialgleichungssystems in Ort und Zeit sowie des Diskretisierens des zugeordneten Phasenraums in Zustände, wobei jedem Zustand ein Gefährdungspotential zugeordnet ist, ferner des Bestimmens von Übergangswahrscheinlichkeiten zwischen Zuständen des diskretisierten Phasenraums, des Erfassens einer ersten Kontamination zu einer ersten Zeit an einem ersten Ort des Leitungsnetzes, des Bestimmens einer Ungenauigkeit der Erfassung der Kontamination, des Bestimmens einer ersten Wahrscheinlichkeitsverteilung von Zuständen des diskretisierten Phasenraums, die der ersten Kontamination und der Unsicherheit zugeordnet ist, des Bestimmens einer zweiten Wahrscheinlichkeitsverteilung, die zeitlich zu der ersten Wahrscheinlichkeitsverteilung versetzt ist, auf der Basis der Übergangswahrscheinlichkeiten, und des Bereitstellens der zweiten Wahrscheinlichkeitsverteilung. Die potentielle Gefährdung kann beispielsweise durch verschmutztes oder mit einem Schadstoff belastetes Wasser erfolgen.

Dadurch ist es möglich, ein Objekt ähnlich einem zellulären Automaten anzugeben, mit dem die Bestimmung vollständig auf diskretisierten Werten stattfindet. Das Differenzialgleichungssystem kann eine oder mehrere Differenzialgleichungen umfassen. Eine der Differenzialgleichungen kann eine Flussreaktion eines im Wasser gelösten kontaminierenden Stoffs repräsentieren. Eine weitere Differenzialgleichung kann den kontaminierenden Stoff modellieren, der an einer Leitungswand anhaftet. Aus der oder den Differenzialgleichungen kann ein numerischer Algorithmus abgeleitet werden, mit dem die Ausbreitung von der Kontamination und ihrer Ungenauigkeit modelliert werden kann. Die Ungenauigkeit kann eine Messungenauigkeit, einen systematischen Fehler oder vollständig unbekannte Einflüsse umfassen. Das vorgeschlagene Konzept basiert auf der Theorie von Transferoperatoren.

Bevorzugterweise werden die Schritte bezüglich des zeitlichen Versatzes iterativ durchgeführt, so dass ein zeitlicher Verlauf der Kontamination bzw. der ihr zugeordneten Wahrscheinlichkeitsverteilung an dem vorbestimmten Ort bestimmt werden kann.

In einer Ausführungsform werden in einer ersten Phase die Übergangswahrscheinlichkeiten auf der Basis des Differenzialgleichungssystems bestimmt und in einer anschließenden, zweiten Phase erfolgt die Bestimmung der zweiten Wahrscheinlichkeitsverteilung auf der Basis der Übergangswahrscheinlichkeiten. Die Bestimmung der Übergangswahrscheinlichkeiten wird also unabhängig von der Bestimmung der zweiten Wahrscheinlichkeitsverteilung durchgeführt.

Dadurch ist es möglich, eine Vorverarbeitung durchzuführen und Regeln zu Zustandsübergängen zu einem Zeitpunkt zu bestimmen, zu dem unter Umständen noch gar kein Messwert für die erste Kontamination vorliegt. Der größte Teil der erforderlichen Bestimmungen kann also vor einer eingetretenen Kontamination durchgeführt werden, wo Rechenzeit keine kritische Größe ist. Dadurch kann auch eine hochauflösende bzw. komplexe Simulation der Ausbreitung einer Kontamination vorbereitet werden, ohne zur Bestimmungszeit die praktischen Grenzen der Berechenbarkeit zu sprengen. Die in der zweiten Phase durchgeführte Bestimmung der zweiten Wahrscheinlichkeitsverteilung kann durch die Vorbereitung in der ersten Phase sehr rasch durchführbar sein. So kann eine schnelle Reaktion auf eine tatsächlich eingetretene Kontamination in einem Leitungsnetz ermöglicht werden.

In einer besonders bevorzugten Ausführungsform umfasst das Diskretisieren der Differenzialgleichung das Erstellen einer Anzahl von Markov-Regeln, wobei das Ableiten der zweiten Wahrscheinlichkeitsverteilung aus der ersten Wahrscheinlichkeitsverteilung auf der Basis der Markov-Regeln erfolgt.

Die Markov-Regeln können einfach als zellulärer Automat (Zustandsübergangssystem) angegeben sein. Für die Abarbeitung von Markov-Regeln existiert eine Vielzahl Hilfsmittel, die eine effiziente Bestimmung unterstützen.

Die Übergangswahrscheinlichkeiten können für eine Stelle des Leitungsnetzes bestimmt und für eine Vielzahl Stellen des Leitungsnetzes verwendet werden. Durch die Modellierung der Entwicklung einer Kontamination an dem einen Ort kann die Bestimmung der Entwicklung an anderen Orten des Leitungsnetzes, die mit dem ersten Ort verbunden sind, insbesondere stromabwärts des ersten Orts, ermöglicht sein.

Die Übergangswahrscheinlichkeit von einem ersten Zustand in einen zweiten Zustand des diskretisierten Phasenraums kann auf der Basis einer Stichprobe für Werte für Kontamination, Ort und Zeit des ersten Zustands und des Differenzialgleichungssystems bestimmt werden.

So können die Übergangswahrscheinlichkeiten mit vertretbarem Aufwand bestimmt werden. Je nach Anforderungen an die Genauigkeit der Modellierung kann die Stichprobe größer oder kleiner gewählt werden.

In einer Ausführungsform modelliert die Differenzialgleichung einen Anteil des kontaminierenden Stoffs, der an der Wand des Leitungsnetzes anhaftet, und einen Anteil des kontaminierenden Stoffs, der mit dem Wasser durch das Leitungsnetz transportiert wird. Dadurch kann die Ausbreitung des kontaminierenden Stoffs durch das Leitungsnetz mit guten Resultaten modelliert sein.

In einer Ausführungsform modelliert die Differenzialgleichung eine Langmuir-Isotherme. Dadurch kann ein übliches und einfaches Sorptionsmodell verwendet werden, das physikalische Grundlagen besitzt. Die Langmuir-Isotherme kann auch als Ausgangsbasis für weitere Adsorptionsmodelle verwendet werden.

Ein Computerprogrammprodukt umfasst Programmcodemittel zur Durchführung des beschriebenen Verfahrens, wobei das Computerprogrammprodukt auf einer Verarbeitungseinrichtung abläuft oder auf einem computerlesbaren Datenträger gespeichert ist.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden, wobei

Figur 1    ein schematisches Leitungsnetz;

Figur 2    ein Zustandsübergangssystem für Gefährdungspotentiale an einer Stelle des Leitungsnetzes von Figur 1;

Figur 3    eine örtliche Erweiterung des Zustandsübergangssystems von Figur 2;

Figur 4    ein Ablaufdiagramm eines Verfahrens zur Bestimmung eines Gefährdungspotentials durch Wasser im Leitungsnetz aus Figur 1;

Figur 5    Ergebnisse einer Modellierung einer Kontaminierung von Wasser im Leitungsnetz von Figur 1, und

Figur 6    Ergebnisse einer weiteren Modellierung analog der Darstellung von Figur 5

darstellt.

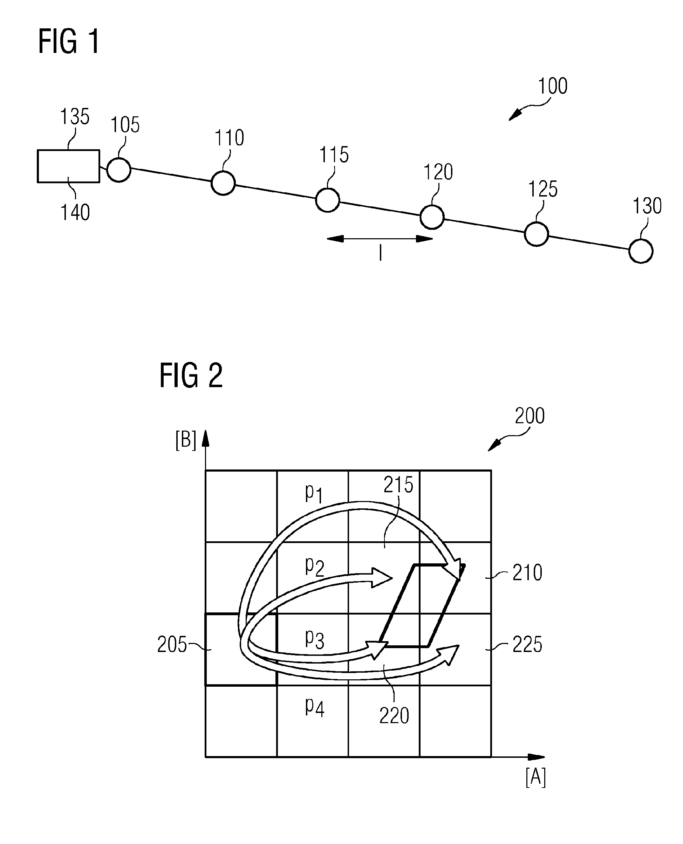

Figur 1 zeigt ein schematisches Leitungsnetz 100, das den folgenden Betrachtungen beispielhaft zugrunde liegt. Das Leitungsnetz 100 umfasst exemplarisch nur eine Leitung, die linear zwischen Stellen 105 bis 130 verläuft. Die Stellen 105 bis 130 können Entnahmestellen, Messpunkte oder virtuelle Punkte repräsentieren. In einer Ausführungsform handelt es sich beim Leitungsnetz 100 um ein Trink- oder Brauchwassernetz, in einer anderen Ausführungsform kann es sich auch um ein Entsorgungsnetz, etwa ein Abwassernetz handeln. Allgemein ist die vorgestellte Technik auch nicht auf den Transport von Wasser im Leitungsnetz 100 beschränkt, obwohl im Folgenden von einem flüssigen Medium ausgegangen wird, in dem sich ein kontaminierender Stoff zu einem gewissen Grad lösen kann.

Abstände zwischen den Stellen 105 bis 130 im Leitungsnetz sind in der Darstellung von Fig. 1 beispielhaft als gleich lang angenommen, wobei die Abstände jeweils die Länge l betragen. Die Flussrichtung verläuft von der Stelle 105 in Richtung der Stelle 130. In der dargestellten, einfachen Ausführungsform sind keine Abzweigungen oder Zuflüsse im Leitungsnetz 100 angenommen. An der Stelle 105 besteht eine Kontaminationsquelle 135. Ein kontaminierender Stoff 140, der aus der Kontaminationsquelle 135 in das Leitungsnetz 100 eintritt, breitet sich mit dem fließenden Wasser über die Stellen 110 bis 125 bis zur Stelle 130 aus.

Mittels eines Differenzialgleichungssystems mit wenigstens einer Differenzialgleichung kann der Transport des kontaminierenden Stoffs 140 aus der Kontaminationsquelle 135 durch das Leitungsnetz 100 modelliert werden. Dazu wird exemplarisch von folgendem Differentialgleichungssystem ausgegangen:

$$\partial_t [A] \pm v \partial_x [A] = f_1 ([A],[B]) \qquad \text{(Gleichung 1)}$$

$$\partial_t [B] = f_2 ([A],[B]) \qquad \text{(Gleichung 2)}$$

Um die aufwändige Verarbeitung des Differentialgleichungssystems zu erleichtern wird vorgeschlagen, eine Konzentration von kontaminierenden Stoffen 140 im Wasser an jedem Ort und für jede Zeit zu diskretisieren, um diskrete Gefährdungspotentiale zu bilden. Außerdem werden die Differenzialgleichungen des Differentialgleichungssystems durch Diskretisierung in eine Anzahl Regeln überführt, die einen Übergang von einem Zustand in einen anderen Zustand modellieren. Die Bestimmung der Ausbreitung der Kontamination erfolgt dann mittels eines Zustandsübergangssystems zwischen Kombinationen von diskreten Parametern.

Ein kontaminierender Stoff 140 im Leitungsnetz 100 kann unterteilt werden in einen ersten Anteil, der im Wasser gelöst ist, das sich im Leitungsnetz 100 befindet, und einen zweiten Anteil, der an der Wand des Leitungsnetzes 100 anhaftet. Mathematisch kann der erste Teil wie in der Gleichung 1 und der zweite Teil wie in der Gleichung 2 modelliert werden.

Dabei können die Funktionen f1 und f2 auch weitere Unsicherheiten modellieren. Sogenanntes weißes Rauschen stellt eine spezielle Unsicherheit dar, die auf diese Weise besonders gut modellierbar ist. Weißes Rauschen ist ein zeitlich nicht korrelierter stochastischer Prozess. Das weiße Rauschen reflektiert Einflüsse, die nicht von einem zeitlich vorherigen Zustand abhängen. Für diese Einflüsse gilt eine maximale Unsicherheit.

Figur 2 zeigt ein Zustandsübergangssystem 200 zur Modellierung von Gefährdungspotentialen an einer der Stellen 105 bis 130 des Leitungsnetzes 100 von Figur 1. In horizontaler Richtung ist der gelöste Anteil des kontaminierenden Stoffs 140 gemäß Gleichung 1 und in vertikaler Richtung der an der Wand des Leitungsnetzes 100 anhaftende Teil gemäß Gleichung 2 angetragen. Beide Angaben sind diskretisiert, stellen also jeweils Bereiche von Konzentrationen des kontaminierenden Stoffs 140 dar.

Durch eine bestimmte Konzentration des gelösten Teils in horizontaler Richtung und eine weitere bestimmte Konzentration des anhaftenden Teils in vertikaler Richtung ist ein erster Zustand 205 gegeben. Dieser Zustand 205 kann mit der Zeit in einen anderen Zustand übergehen.

Die Bestimmung der Kontamination im Leitungsnetz 100 unterliegt jedoch stets einer gewissen Unsicherheit. Die Unsicherheit kann aus einer Kontaminationsquelle, einer Messmethode, einem Messfehler, einem Ablesefehler, einem Übertragungsfehler oder einem anderen Einfluss resultieren.

Ein Übergang vom ersten Zustand in einen anderen Zustand kann üblicherweise nicht konkret angegeben werden, vielmehr bestehen unterschiedliche Wahrscheinlichkeiten für Übergänge in andere Zustände. In der Darstellung von Figur 2 erfolgt, ausgehend vom ersten Zustand 205 ein Übergang in einen zweiten Zustand 210 mit einer Wahrscheinlichkeit p1, in einen dritten Zustand 215 mit einer Wahrscheinlichkeit p2, in einen vierten Zustand 220 mit einer Wahrscheinlichkeit p3 und in einen fünften Zustand 225 mit einer Wahrscheinlichkeit p4. Dabei sind rein exemplarisch vier weitere Zustände 210 - 225 mit den zugeordneten Übergangswahrscheinlichkeiten p1 - p4 angenommen. Die Übergänge und Wahrscheinlichkeiten werden mit Hilfe der Funktionen f1 und f2 der Gleichungen 1 und 2 bestimmt. So kann zum Zustand 205 nicht nur ein Folgezustand, sondern eine Vielzahl Folgezustände mit zugeordneten Wahrscheinlichkeiten angegeben sein.

Praktisch liegt jedoch nicht nur ein Zustand 205, sondern eine Wahrscheinlichkeitsverteilung mehrerer Zustände 205 vor, die mit der Zeit in Wahrscheinlichkeitsverteilungen anderer Zustände übergehen können.

In der Praxis können die Übergangswahrscheinlichkeiten p1 - p4 bestimmt werden, indem Werte einer Stichprobe von Kontaminationen des ersten Zustands 205 in das Differenzialgleichungssystem der Gleichungen 1 und 2 eingesetzt werden, um jeweilige Folgezustände 210 - 225 zu bestimmen. Die Häufigkeiten der Übergänge in die Zustände 210 - 225 können dann in bekannter Weise in die Wahrscheinlichkeiten p1 - p4 umgerechnet werden. Dabei wird die Angabe der Wahrscheinlichkeiten p1 - p4 umso genauer, je größer die Stichprobe ist.

Die bestimmten Übergangswahrscheinlichkeiten p1 - p4 zwischen den Zuständen 205 bis 225 können als Markov-Regeln ausgedrückt werden, um eine nachfolgende Verarbeitung bestimmter Kontaminationen zu erleichtern.

Ein Zustand 210-225 ist also vom vorhergehenden Zustand 205 abhängig und ein Übergang von einem Zustand 205 in einen Folgezustand 210-225 hat eine zugewiesene Wahrscheinlichkeit p1 - p4.

Ein Beispiel für ein Differenzialgleichungssystem im Sinne der Gleichungen 1 und 2 bietet die sogenannte Langmuir-Isotherme. Die Langmuir-Isotherme gilt als das einfachste Sorptionsmodell, das physikalische Grundlagen besitzt. Es werden die Annahmen getroffen, dass

- Adsorption in einer einzelnen molekularen Schicht stattfindet,
- alle Sorptionsplätze gleichwertig sind und die Oberfläche gleichförmig ist,
- es keine Wechselwirkungen zwischen benachbarten Sorptionsplätzen und den adsorbierten Teilchen gibt.

Die Langmuir-Isotherme kann eine maximale Beladung der Sorptionsoberflächen abbilden und ist damit Ausgangsbasis für weitere Adsorptionsmodelle.

Wird ein vorbestimmter Abschnitt des Leitungsnetzes 100 betrachtet, beispielsweise zwischen den Stellen 105 und 110, so kann der gelöste Anteil des kontaminierenden Stoffs 140 gemäß Gleichung 1 angegeben werden als:

$$\partial_t D + v \partial_x D = -\frac{1}{r_h} \cdot \frac{1}{\frac{1}{k_1} + \frac{1}{k_f}(S_{\max} - A)} \cdot \left( D(S_{\max} - A) - K_{eq} A \right) \qquad \text{(Gleichung 5)}$$

Der an den Wänden des Leitungsnetzes 100 anhaftende Anteil des kontaminierenden Stoffs 140 gemäß Gleichung 2 kann dann so ausgedrückt werden:

$$\partial_t [A] = \frac{1}{\frac{1}{k_1} + \frac{1}{k_f}(S_{\max} - A)} \cdot \left( D(S_{\max} - A) - K_{eq} A \right) \qquad \text{(Gleichung 6)}$$

Für die Gleichungen 5 und 6 gilt:

$S_{\max}$    maximale Kapazität des Leitungsnetzes für den adsorbierten kontaminierenden Stoff 140

$K_{eq}$    Gleichgewichtskonstante

$k_1$    Adsorptionskonstante

$k_f$    Massentransport Koeffizient

$r_h$    hydraulischer Leitungsradius; definiert als Quotient aus Leitungsvolumen und Leitungsoberfläche

$v$    Fließgeschwindigkeit des Mediums in der Leitung

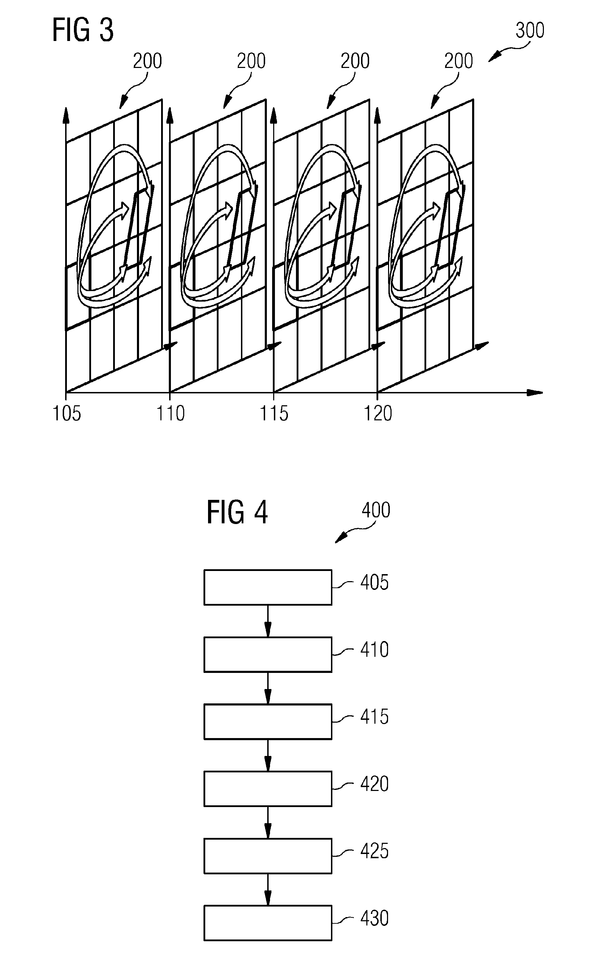

Figur 3 zeigt eine Erweiterung 300 des Zustandsübergangsystems von Figur 2 bezüglich des Orts der Kontamination. Dabei werden die bezüglich einer der Stellen 105 - 130 bestimmten Übergangswahrscheinlichkeiten p1 - p4 unverändert für die restlichen Stellen 105 - 130 verwendet.

In der Darstellung von Figur 3 ist in horizontaler Richtung ein Ort angetragen, wobei die Stellen 105 bis 120 des Leitungsnetzes 100 aus Figur 1 zur Orientierung angegeben sind. Eine Darstellung für die Stellen 125 und 130 wurden aus Übersichtlichkeitsgründen weggelassen. An jeder der Stellen 105 bis 120 ist ein Zustandsübergangssystem 200 entsprechend der Darstellung von Figur 2 eingezeichnet. In dieser Darstellung wird deutlich, wie sich benachbarte Zustandsübergangssysteme 200 aufgrund der topologischen Anordnung der ihnen zugeordneten Stellen 105 - 130 beeinflussen können. Diese Beeinflussung kann ebenfalls durch die Funktionen f1 und f2 modelliert sein.

Figur 4 zeigt ein Ablaufdiagramm eines Verfahrens 400 zur Bestimmung eines Gefährdungspotentials durch Wasser im Leitungsnetz 100 aus Figur 1. In einem ersten Schritt 405 werden Konzentrationen eines kontaminierenden Stoffs diskretisiert. Im Einzelnen bedeutet dies, dass Bereiche zu erwartender Konzentrationen eines kontaminierenden Stoffs 140 diskreten Gefährdungspotentialen zugeordnet werden. Diese Unterteilung wird üblicherweise anhand eines schädigenden Potentials des kontaminierenden Stoffs 140 auf einen Mensch bestimmt, der mit kontaminiertem Wasser aus dem Leitungsnetz 100 in Berührung kommt. Beispielsweise würden Konzentrationen von Arsenat im Wasser andere Schwellenwerte zwischen den Risikoklassen erfordern als etwa Jod oder Colibakterien.

In einem folgenden Schritt 410 wird ein Differenzialgleichungssystem, das die Ausbreitung einer Kontamination im Leitungsnetz 100 beschreibt, erfasst. Eine oder mehrere Differenzialgleichungen, die das Differenzialgleichungssystem bilde, werden bezüglich der Kontamination, des Orts und der Zeit diskretisiert. Beispielsweise können die Gleichungen 1 und 2 exemplarisch für Differenzialgleichungen eines Differenzialgleichungssystems verwendet werden. Durch die Diskretisierung kann im Folgenden eine Bestimmung auf Risikoklassen statt beispielsweise auf Konzentrationen des Stoffs durchgeführt werden.

Mit der Diskretisierung der Differenzialgleichung verbunden ist ein Schritt 415, in welchem Wahrscheinlichkeiten für Übergänge zwischen Zuständen 205 - 225 des diskretisierten Phasenraums aus Ort und Kontamination bestimmt werden. Diese Übergangswahrscheinlichkeiten p1 - p4 können, wie oben mit Bezug auf Figur 2 bereits beschrieben wurde, auf der Basis einer Stichprobe bestimmt werden, deren Werte in das Differenzialgleichungssystem eingesetzt werden um mögliche Folgezustände zu bestimmen. In einer Ausführungsform werden die Übergangswahrscheinlichkeiten p1 - p4 als Regeln einer Markov-Kette ausgedrückt. Damit ist eine Vorverarbeitung des Modells abgeschlossen.

In einem Schritt 420 wird eine Kontamination des Leitungsnetzes 100, beispielsweise an der Stelle 105, erfasst. Der erfasste Wert stammt beispielsweise aus einer Modellannahme, einer Einschätzung oder einer chemischen bzw. physikalischen Messung und betrifft eine Konzentration des kontaminierenden Stoffs 140. Eine Unsicherheit, mit der diese Messung naturgemäß behaftet ist, wird in diesem Schritt ebenfalls erfasst.

Auf der Basis der Kontamination und der Unsicherheit wird in einem Schritt 425 eine erste Wahrscheinlichkeitsverteilung von Zuständen des diskretisierten Phasenraums bestimmt. Diese Verteilung drückt aus, mit welcher Wahrscheinlichkeit zum Betrachtungszeitpunkt am Betrachtungsort welche Klasse von Gefährdungspotential vorlag.

Anschließend wird in einem Schritt 430 eine zweite Wahrscheinlichkeitsverteilung, die zeitlich zu der ersten Wahrscheinlichkeitsverteilung versetzt ist, auf der Basis der ersten Wahrscheinlichkeitsverteilung und der Übergangswahrscheinlichkeiten p1 - p4 bestimmt. Diese Bestimmung wird bevorzugterweise mittels der Regeln durchgeführt, die in Schritt 415 erstellt wurden. Die bestimmte zweite Wahrscheinlichkeitsverteilung wird als Ergebnis bereitgestellt. Anschließend kann das Verfahren 400 iterativ zum Schritt 430 zurückkehren, um eine dritte Wahrscheinlichkeitsverteilung auf der Basis der zweiten Wahrscheinlichkeitsverteilung und der Übergangswahrscheinlichkeiten p1 - p4 zu bestimmen und so fort.

Von besonderem Vorteil des Verfahrens 400 ist, dass die Schritte 405 bis 415 unabhängig von den Schritten 420 bis 430 durchgeführt werden können. Insbesondere können die Übergangswahrscheinlichkeiten p1 - p4, mit denen Übergänge zwischen den Zuständen 205 bis 225 stattfinden können, für eine oder mehrere der Stellen 105 bis 130 bereits bestimmt werden, noch bevor im Schritt 420 die Kontamination erfasst wurde. Dadurch ist es möglich, für die Erstellung der Regeln einen großen rechentechnischen Aufwand zu betreiben, ohne dass die Bestimmung der zweiten Wahrscheinlichkeitsverteilung nach dem Feststellen der Kontamination bzw. nach dem Bestimmen der ersten Wahrscheinlichkeitsverteilung im Schritt 420 zeitintensiv wäre. Anders ausgedrückt kann in den Schritten 405 bis 420 ein Modell vorprozessiert werden, auf dessen Basis anschließend bei Vorliegen einer Kontamination mit hoher Geschwindigkeit deren Verhalten bzw. Ausbreitung bestimmt werden kann.

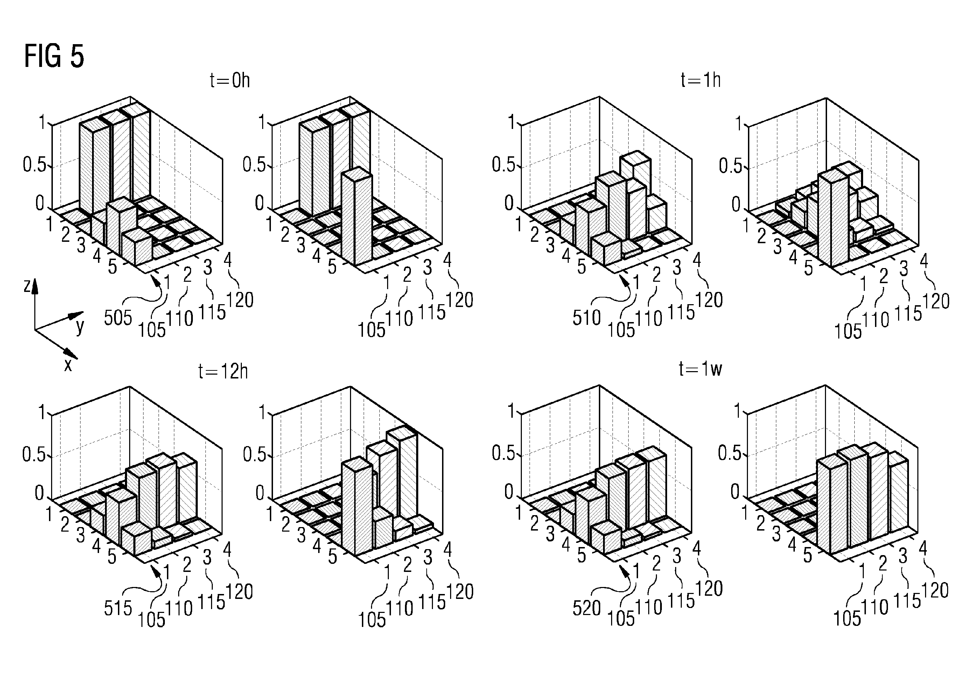

Figur 5 zeigt Ergebnisse einer beispielhaften Modellierung einer Kontaminierung von Wasser im Leitungsnetz 100 von Figur 1 durch Arsenat. Dieser Modellierung liegt die Langmuir-Isotherme der Gleichungen 5 und 6 zu Grunde. Insgesamt sind acht dreidimensionale Diagramme dargestellt, die paarweise einem Zeitpunkt zugeordnet sind. Die beiden linken Diagramme in der oberen Zeile sind dem Zeitpunkt t = 0 Stunden, die beiden rechten in der gleichen Zeile im Zeitpunkt t = 1 Stunde zugeordnet. In der unteren Zeile sind die beiden linken Diagramme dem Zeitpunkt t = 12 Stunden und die beiden rechten dem Zeitpunkt t = 1 Woche zugeordnet. Für jeden Zeitpunkt stellt das jeweils linke Diagramm einen gelösten Anteil von Arsenat gemäß Gleichung 1 und das rechte Diagramm an der Wand des Leitungsnetzes 100 anhaftendes Arsenat gemäß Gleichung 2 dar.

Jedes der acht Diagramme erstreckt sich entlang dreier Achsen x, y und z. Die x-Achse zeigt Gefährdungspotentiale, die y-Achse Stellen 105 bis 120 und die z-Achse Wahrscheinlichkeiten. Dabei sind die Gefährdungspotentiale durch Klassifizierung von Kontaminationen bzw. Konzentrationen von Arsenat diskret angegeben. Die Stellen 105 - 120 sind naturgemäß ebenfalls diskret angegeben, während die Wahrscheinlichkeiten undiskretisiert im Intervall zwischen 0 und 1 angegeben sind.

Jede in der x-z-Ebene liegende Darstellung in jedem der acht Diagramme repräsentiert eine Wahrscheinlichkeitsverteilung von Gefährdungspotentialen, die an der entsprechenden Stelle 105 - 130 zur entsprechenden Zeit t vorliegt. Exemplarisch sind erste bis vierte Wahrscheinlichkeitsverteilungen 505 bis 520 angegeben.

Für die vorliegende Darstellung wird davon ausgegangen, dass die Kontaminationsquelle 135 an der Stelle 105 fortwährend einen konstanten Strom von Arsenat in das Leitungsnetz 100 entlässt. Dabei ist sowohl der Strom von Arsenat als auch dessen Kontinuität über die Zeit einer gewissen Unsicherheit unterworfen.

Zum Zeitpunkt t = 0 Stunden zeigt sich für das gelöste Arsenat für die Stelle 105 ein weißes Rauschen der Wahrscheinlichkeiten der Gefährdungspotentiale entsprechend einer Gauß'schen Glockenkurve. Für die Stellen 110 bis 120 besteht mit der höchstmöglichen Wahrscheinlichkeit die geringstmögliche Gefährdung. Im rechten Diagramm ist erkennbar, dass an der Stelle 105 mit maximaler Wahrscheinlichkeit eine maximale Menge Arsenat an der Wand der Leitung adsorbiert ist.

Für die Folgezeitpunkte t = 1 Stunde, 12 Stunden oder 1 Woche kann gesehen werden, wie sich das Arsenat entlang des Leitungsnetzes 100 in Richtung der Stellen 110 bis 120 ausbreitet. Nach einer Woche befindet sich ungelöstes Arsenat mit sehr hohen Wahrscheinlichkeiten an allen Stellen 105 bis 120. Die Verteilung von gelöstem Arsenat begründet mit hoher Wahrscheinlichkeit noch das zweithöchste Gefährdungspotential.

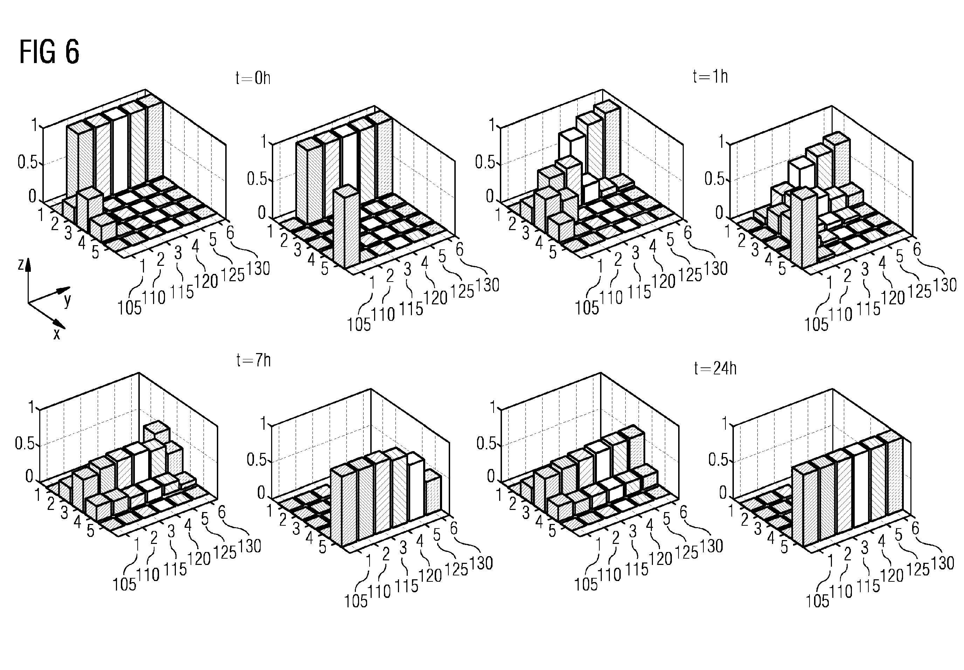

Figur 6 zeigt Ergebnisse einer weiteren Modellierung analog der Darstellung von Figur 5. Diese Darstellung berücksichtigt alle Stellen 105 bis 130 im Leitungsnetz 100 aus Figur 1 und ist ansonsten so aufgebaut wie Figur 5. Die betrachteten Zeitpunkte betragen t=0 Stunden für die Darstellungen oben links, t=1 Stunde für die Darstellungen oben rechts, t= 7 Stunden für die Darstellungen unten links und t= 24 Stunden für die Darstellungen unten rechts. Dieser Simulation liegen andere Ausbreitungsparameter der Kontamination zu Grunde als der Simulation von Figur 5. Auch hier lässt sich die allmähliche Ausbreitung des Arsenats 140 durch das Leitungsnetz 100 und sein Löseverhalten gut ablesen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren (400) zur Bestimmung eines Gefährdungspotentials durch eine Kontamination in einem Leitungsnetz (100), wobei das Verfahren (400) folgende Schritte umfasst:

   - Erfassen (410) eines partiellen Differenzialgleichungssystems zur Beschreibung einer Entwicklung einer Kontamination im Leitungsnetz (100);
   - Diskretisieren (410) des Differenzialgleichungssystems in Ort und Zeit und Diskretisieren des zugeordneten Phasenraums in Zustände, wobei jedem Zustand (205 - 225) ein Gefährdungspotential zugeordnet ist;
   - Bestimmen (415) von Übergangswahrscheinlichkeiten (p1 - p4) zwischen Zuständen (205 - 225) des diskretisierten Phasenraums;
   - Erfassen (420) einer ersten Kontamination zu einer ersten Zeit an einem ersten Ort des Leitungsnetzes;
   - Bestimmen (420) einer Ungenauigkeit der Erfassung der Kontamination;
   - Bestimmen (425) einer ersten Wahrscheinlichkeitsverteilung (505) von Zuständen (205 - 225) des diskretisierten Phasenraums, die der ersten Kontamination und der Unsicherheit zugeordnet ist;
   - Bestimmen (430) einer zweiten Wahrscheinlichkeitsverteilung (510), die zeitlich zu der ersten Wahrscheinlichkeitsverteilung (505) versetzt ist, auf der Basis der Übergangswahrscheinlichkeiten, und
   - Bereitstellen (430) der zweiten Wahrscheinlichkeitsverteilung (510).

2. Verfahren (400) nach Anspruch 1, wobei die Schritte bezüglich des zeitlichen Versatzes iterativ durchgeführt werden (425, 430).

3. Verfahren (400) nach Anspruch 1 oder 2, wobei in einer ersten Phase (405-415) die Übergangswahrscheinlichkeiten auf der Basis des Differenzialgleichungssystems bestimmt werden und in einer anschließenden zweiten Phase (420-430) die Bestimmung der zweiten Wahrscheinlichkeitsverteilung (510) auf der Basis der Übergangswahrscheinlichkeiten (p1 - p4) erfolgt.

4. Verfahren (400) nach einem der vorangehenden Ansprüche, wobei das Bestimmen (415) der Übergangswahrscheinlichkeiten das Erstellen einer Anzahl Markov-Regeln umfasst und das Ableiten (430) der zweiten Wahrscheinlichkeitsverteilung (510) aus der ersten Wahrscheinlichkeitsverteilung (505) auf der Basis der Markov-Regeln erfolgt.

5. Verfahren (400) nach einem der vorangehenden Ansprüche, wobei die Übergangswahrscheinlichkeiten für eine Stelle (105-130) des Leitungsnetzes bestimmt und für eine Vielzahl Stellen (105-130) des Leitungsnetzes verwendet werden.

6. Verfahren (400) nach einem der vorangehenden Ansprüche, wobei die Übergangswahrscheinlichkeit von einem ersten Zustand in einen zweiten Zustand des diskretisierten Phasenraums auf der Basis einer Stichprobe für Werte für Kontamination, Ort und Zeit des ersten Zustands und des Differenzialgleichungssystems bestimmt (415) wird.

7. Verfahren (400) nach einem der vorangehenden Ansprüche, wobei das Differenzialgleichungssystem einen Anteil des kontaminierenden Stoffs (140), der an der Wand des Leitungsnetzes (100) anhaftet, und einen Anteil des kontaminierenden Stoffs (140), der mit dem Wasser durch das Leitungsnetz (100) transportiert wird, modelliert.

8. Verfahren (400) nach Anspruch 7, wobei das Differenzialgleichungssystem eine Langmuir-Isotherme modelliert.

9. Computerprogrammprodukt mit Programmcodemitteln zur Durchführung eines Verfahrens (400) nach einem der vorangehenden Ansprüche, wobei das Computerprogrammprodukt auf einer Verarbeitungseinrichtung abläuft oder auf einem computerlesbaren Datenträger gespeichert ist.

# FIG 1

# FIG 2

## FIG 3

300

200     200     200     200

105     110     115     120

## FIG 4

400

405

410

415

420

425

430

FIG 5

t=0h

t=1h

t=12h

t=1w

FIG 6

EP 2 674 873 A2